# EUROPEAN PATENT APPLICATION

(11) **EP 3 841 893 A1**
(43) Date of publication of application: **30.06.2021**
(21) Application number: 19852579.2
(22) Date of filing: 22.08.2019
(51) Int. Cl.: A23L 33/105, A61K 36/282, A61P 31/18

(54) **COMPOSITION FOR PREVENTING TRANSMISSION OF HUMAN IMMUNODEFICIENCY VIRUS**

(30) Priority: 23.08.2018 JP 2018168312
(71) Applicant: Matsuda, Tomotake, Tokyo 106-0032 (JP); Nakamura, Takao, Tokyo 133-0065 (JP)
(72) Inventor: Matsuda, Tomotake, Tokyo 106-0032 (JP); Nakamura, Takao, Tokyo 133-0065 (JP)
(74) Representative: Pfenning, Meinig & Partner mbB
(86) International application number: PCT/JP2019/032736
(87) International publication number: WO 2020/040236

(57) **Abstract**

An aspect of the present invention is a composition containing an extract of Artemisia absinthium. The composition is one for preventing a human body from being infected with a human immunodeficiency virus (HIV). The extract may be extracted by using an extracting medium containing more than 57.5 vol.% alcohol. The extracting medium may contain at least one of a monohydric alcohol and a dihydric alcohol as a main component. The composition may be a microbicide selected from a group consisting of an aqueous solution, a gel, a sol, a cream, and a suppository. The composition may be one to be applied to a skin or a mucous membrane. Another aspect of the present invention is an implement that contains the composition and is applied to a surface of a body.

## Description

### Technical Field

The prevent invention relates to a composition for preventing infection with a human immunodeficiency virus (HIV).

### Background Art

Non-patent Literature 1 discloses a composition for preventing HIV infection, i.e., a microbicide.

(Excerpts from Original Text) "Microbicides are compounds that can be applied inside the vagina or rectum to protect against sexually transmitted infections (STIs) including HIV. They can be formulated as gels, creams, films, or suppositories. Microbicides may or may not have spermicidal activity (contraceptive effect). At present, an effective microbicide is not available."

(Japanese Translation) "Microbicides are compounds that can be applied inside the vagina or rectum to protect against sexually transmitted infections (STIs) including HIV. They can be formulated as gels, creams, films, or suppositories. Microbicides may or may not have spermicidal activity (contraceptive effect). At present, an effective microbicide is not available."

Patent Literature 1 discloses a virus adsorbent containing sacran as an active component. The sacran is a substance extracted from Aphanothece sacrum. A composition containing this virus adsorbent can be used as a microbicide for HIV.

Patent Literatures 2 to 5 disclose various uses of extracts from Artemisia absinthium which the inventors of the present invention referred to.

### Citation List

### Patent Literature

Patent Literature 1: Japanese Unexamined Patent Application Publication No. 2018-123110
Patent Literature 2: Japanese Unexamined Patent Application Publication No. 2013-010728
Patent Literature 3: Japanese Unexamined Patent Application Publication No. 2013-010729
Patent Literature 4: Japanese Unexamined Patent Application Publication No. 2017-105712
Patent Literature 5: Published Japanese Translation of PCT International Publication for Patent Application, No. 2006-504787

### Non Patent Literature

Non-patent Literature 1: World Health Organization, "Microbicides", [online], WHO> WHO sites> HIV/AIDS> HIV/AIDS Topics, [Searched on July 30, 2019], the Internet <URL: https://www.who.int/hiv/topics/ microbicides/microbicides/en/>

### Summary of Invention

### Technical Problem

The prevent invention provides a composition for preventing HIV infection.

### Solution to Problem

<1> A composition containing an extract of Artemisia absinthium, for preventing a human body from being infected with a human immunodeficiency virus.
<2> The composition described in Item <1>, in which the extract is extracted by using an extracting medium containing more than 57.5 vol.% alcohol.
<3> The composition described in Item <2>, in which the extracting medium contains at least one of a monohydric alcohol and a dihydric alcohol as a main component.
<4> The composition described in Item <2>, in which
   the extracting medium contains 98 vol.% or more ethanol,
   the rest comprises water, and
   the extraction is carried out by distillation.
<5> The composition described in Item <2>, in which
   the extracting medium is 100% of 1,3-butylene glycol, and
   the extraction is carried out by leaching.
<6> The composition described in any one of Items <1> to <5>, in which the composition is a microbicide selected from a group consisting of an aqueous solution, a gel, a sol, a cream, and a suppository.
<7> The composition described in Item <6>, in which the composition is one to be applied to a skin or a mucous membrane.
<8> The composition described in Item <6>, in which the composition is one to be applied to a penis of a human, or inside of a vagina or a vulva of a human.
<9> An implement including a composition described in any one of Items <1> to <5>, the implement being adapted to be applied to a surface of a body.
<10> A condom or a dental dam coated with a composition described in any one of Items <1> to <5>.

### Advantageous Effects of Invention

According to the prevent invention, it is possible to provide a composition for preventing HIV infection.

### Description of Embodiments

In an embodiment, Artemisia absinthium is used as an ingredient in order to obtain a composition for preventing infection with a human immunodeficiency virus (HIV). This composition is useful to provide, for example, a microbicide targeted for HIV. The HIV, which is the target of the microbicide, is at least one of HIV-1 and HIV-2 both of which belong to the genus Lentivirus in the family Retroviridae.

### <Ingredient>

Artemisia absinthium has been used as an ingredient for liqueurs from ancient times. A liqueur made from Artemisia absinthium is called absinthe. In Japan, Artemisia absinthium has been used as a food additive for giving a bitter taste to foods. In recent years, Artemisia absinthium has been added in cosmetics for preserving them, or enhancing the preservation thereof (Patent Literature 2). Further, since Artemisia absinthium suppresses the growth of fungi, it is added in products for maintaining a hygienic environment (Patent Literature 3).

Further, an extraction liquid (a liquid state extract) of Artemisia absinthium serves as a proliferation inhibitor for viruses of the family Flaviviridae, including Hepatitis C Virus, Bovine Viral Diarrhea Virus, Classical Swine Fever Virus, Yellow Fever Virus, and Dengue Virus (Patent Literature 4). Further, components contained in Artemisia absinthium, such as artemisinin, alleviate symptoms caused by these viruses (Patent Literature 5).

These literatures do not disclose that Artemisia absinthium or components contained in Artemisia absinthium, such as artemisinin and analogs thereof, suppress HIV or alleviate symptoms developed by HIV, i.e., alleviate an acquired immunodeficiency syndrome (AIDS).

Artemisia absinthium is a plant belonging to the genus Artemisia in the family Compositae. The genus Artemisia in the family Compositae also includes Artemisia princeps Pampanini (Artemisia folium, Artemisia princeps), Artemisia capillaris, Artemisia vulgaris, and Artemisia montana. They are closely related to Artemisia absinthium, but are not the same species as that of Artemisia absinthium.

### <Dried Substance>

The anti-HIV activity of a composition according to this embodiment is derived from Artemisia absinthium. The active component of the composition according to this embodiment suppresses the activity of HIV. In an aspect, the active component is a dried substance using a part of, or two or more parts of a leaf, a stem, a root, and a flower of Artemisia absinthium. In an aspect, the dried substance of Artemisia absinthium is obtained by drying, after harvesting Artemisia absinthium, these parts thereof for 72 hours or longer.

### <Extract>

In an aspect, the active component of the composition is an extract of Artemisia absinthium. In an aspect, the extract is extracted from a part of, or two or more parts of a leaf, a stem, a root, and a flower of Artemisia absinthium by using a suitable extracting medium. In an aspect, the extract is extracted from the whole plant of Artemisia absinthium. In an aspect, the above-described dried substance of Artemisia absinthium is used for the extraction. In an aspect, the extract may be a crude extract that has not been purified yet.

An example of the extraction method is leaching from an ingredient into an extracting medium. In an aspect, the temperature in the extraction is a room temperature of 15 to 25°C. In an aspect, the extraction time is no shorter than 24 hours and no longer than 72 hours.

Another example of the extraction method is distillation. In an aspect, the extraction is carried out by heating and distilling an extracting medium together with an ingredient(s), i.e., heating and distilling an extracting medium in which an ingredient(s) is immersed. In an aspect, the temperature in the extraction is from a room temperature to 100°C. Note that the room temperature is 15 to 25°C. In an aspect, the extraction time is no shorter than two hours and no longer than six hours.

In an aspect, the extracting medium is composed of one of, or two or more of water, alcohols, aliphatic hydrocarbons, unsaturated hydrocarbons, aromatic hydrocarbons, alkanes, dihydric alcohols, trihydric alcohols, polyhydric alcohols, aliphatic alcohols, acetic acid and acetic esters, ethylene oxides, propylene oxides, copolymers of ethylene oxides and propylene oxides, aliphatic ketones, plant-derived fatty oils, honey, insect secretions, animal secretions, animal-derived fatty oils, plant extracts, animal extracts, microbial extracts, raw-sugar extracts, seawater, and other solvents.

In an aspect, the extract is extracted from Artemisia absinthium by using an extracting medium containing an alcohol(s). In an aspect, the content of the alcohol in the extracting medium is larger than 57.5 vol.%. Further, the rest of the extracting medium comprises water. In an aspect, the content of the alcohol in the extracting medium is 60, 70, 80, 90, or 100 vol.%. In an aspect, the extracting medium contains at least one of a monohydric alcohol and a dihydric alcohol as a main component. In an aspect, the extracting medium contains 98 vol.% or more ethanol. In an aspect, the extracting medium is 100% of 1,3-butylene glycol.

The composition may be obtained by mixing the extract with another component(s). In an aspect, the extract may be in a dissolved state in which the extract is dissolved in the extracting medium. That is, the extraction liquid may be mixed with another component(s). In an aspect, the extract which is separated from the extracting medium may be mixed with another component(s). That is, a component(s) of the extract may be isolated and purified from the extraction liquid, and then mixed with another component(s).

### <Microbicide>

In an aspect, HIV infection is prevented by bringing, beforehand, the microbicide into contact with cells that have not been infected with HIV. In an aspect, HIV infection is prevented by bringing the microbicide into direct contact with cells that are infected with HIV.

The microbicide according to this embodiment is a composition containing the above-described extract of Artemisia absinthium. Examples of its dosage form contain an aqueous solution, a gel, a sol, a cream, and a suppository. An aspect of the aqueous solution is a lotion having viscosity and lubricity. An aspect of the gel is a jelly having lubricity.

In an aspect, the microbicide contains an additive. Examples of the additive in the form of the aqueous solution contain water-soluble low molecular-weight substances and water-soluble high molecular-weight substances (i.e., water-soluble polymers). Examples of the water-soluble low molecular-weight substance include ethanol, ethylene glycol, and glycerol. The other additives include moisturizers, colorants, fragrances, and algefacient. Further, an oily additive imparts lubricity to the microbicide before and after the application thereof.

The microbicide is locally applied to any part of a body. In an aspect, the microbicide is applied to a skin. In an aspect, the microbicide is applied to a penis and the like of a human. In an aspect, the microbicide is applied to the inside of a vagina, a vulva, and the like of a human.

In an aspect, the microbicide is applied to a mucous membrane. The mucous membrane is an epithelial layer derived from ectoderms, covered by epithelial cells. The mucous membrane is involved in absorption and secretion. A mucous membrane is present in various body cavities, and is exposed to an external environment or is in contact with an internal organ. A mucous membrane is present in nostrils, lips, ears, genitals, and an anus, and is connected to a skin in various places.

### <Implement>

An aspect of the embodiment is an implement including the above-described composition. The implement is applied to a surface of a body. The implement may be a film. The implement may be a film coated with the composition. The implement may be a condom or a dental dam coated with the composition.

### [Example]

The anti-HIV activities of extracts of Artemisia absinthium were evaluated by using MT-4 cells derived from human T-cells that were positive to human T-cell leukemia virus type 1 (HTLV-1).

Firstly, MT-4 cells were divided into a group of cells that were infected with an HIV-1 IIIB strain and a group of cells that were not infected therewith. In the infected group, the HIV-1 IIIB strain was added to the MT-4 cells at a concentration of 0.002 moles. In the infected group, the MT-4 cells were infected with the HIV-1 IIIB when no inhibition was made. The HIV-1 IIIB strain was not added in the non-infected group. Next, an extraction liquid of Artemisia absinthium was added to each of the groups, and their anti-HIV activities were evaluated. Azidothymidine (AZT), i.e., a nucleoside analogue reverse transcriptase inhibitor of a HIV therapeutic medicine was added in the positive control group.

The extraction liquid was obtained from a dried substance of the whole plant. The extracting mediums were ethanol, 1,3-butylene glycol, and a mixed liquid of 1,3-butylene glycol and propanediol. An extract obtained by using each of the extracting mediums was added to MT-4 cells at each of concentrations of 0.00%, 0.10%, 0.20%, 0.39%, 0.78%, 1.56%, 3.13%, 6.25%, 12.5%, 25.0% and 50.0%. The concentrations are expressed by vol.%.

Further, for the positive control group, AZT was added to MT-4 cells at each of concentrations of 0.0 nM, 0.2 nM, 0.4 nM, 0.8 nM, 1.6 nM, 3.1 nM, 6.3 nM, 13.0 nM, 25.0 nM, 50.0 nM, and 100.0 nM.

The AZT exhibited an anti-HIV activity in the positive control group in a concentration-dependent manner. Therefore, it was confirmed that the testing system worked.

Viable cells of the MT-4 cells infected with the HIV-1 IIIB strain exhibited a yellowish-brown color. It is possible to evaluate the anti-HIV activity of the extract of Artemisia absinthium by measuring the color tone based on the absorbance.

The examples of experiments, which were divided according to the extracting medium, will be described hereinafter one by one.

### <Example 1>

An extraction was carried out for a dried substance, which was obtained by drying the whole plant of Artemisia absinthium, by using 1,3-butylene glycol. The temperature in the extraction was a room temperature of 15 to 25°C. The extraction time was no shorter than 24 hours and no longer than 72 hours. The extraction method was leaching from the ingredient to the extracting medium.

When the concentration of the extraction liquid was 1.56% or higher, 75% or more of uninfected viable cells were observed. Note that, the viable cell count in the sample of HIV non-infected group to which an extraction liquid having the same concentration was added was set to be 100%. The liquid extracted by 1,3-butylene glycol exhibited an anti-HIV activity of about 75% at a concentration of 1.56%.

It is also conceivable that HIVs were killed by the cytotoxicity of the liquid extracted by 1,3-butylene glycol. This idea (i.e., the reasoning) is based on the fact that the anti-HIV activity was observed at the additive concentration of 1.56%.

### <Example 2>

An extraction was carried out for a dried substance, which was obtained by drying the whole plant of Artemisia absinthium, by using a 98% aqueous solution of ethanol. The ratio of ethanol in the extracting medium is expressed by vol.%. The temperature in the extraction was 100°C. The extraction time was no shorter than two hours and no longer than four hours. The extraction method was direct distillation, i.e., the so-called ethanol distillation. The extraction was carried out by heating and distilling the extracting medium together with the ingredient(s), i.e., heating and distilling the extracting medium in which the ingredient(s) was immersed.

When the concentration of the extraction liquid was 0.2%, 30% or more of viable cells were observed. Note that, the viable cell count in the sample of HIV non-infected group to which an extraction liquid having the same concentration was added was set to be 100%. The liquid extracted by ethanol exhibited an anti-HIV activity of about 30% at a concentration of 0.2%.

It is conceivable that the liquid extracted by ethanol had an anti-HIV activity that also affected the entity of HIV. This is because the anti-HIV activity by the liquid extracted by ethanol exhibited an increasing tendency, which started at an additive concentration of 0.10%, in a concentration-dependent manner.

### <Example 3>

An extraction was carried out for a dried substance, which was obtained by drying the whole plant of Artemisia absinthium, by using a mixed extracting medium containing 50% of propanediol and 7.5% of 1,3-butylene glycol. The rest, which was contained in 42.5% in the extracting medium, was water. The ratio of each component in the extracting medium is expressed by vol.%. The temperature in the extraction was a room temperature of 15 to 25°C. The extraction time was no shorter than 24 hours and no longer than 72 hours. The extraction method was leaching from the ingredient to the extracting medium.

No or little anti-HIV activity was observed when the concentration of the extraction liquid was 50.0% or lower.

### <Discussion>

In the Example 3, the extracting medium contained 57.5% of an organic medium and 42.5% of water. When this extracting medium is compared with those used in the Examples 1 and 2, the content of the organic medium was about half of those in the Examples 1 and 2. Therefore, it is expected that the extraction liquids of the Examples 1 and 2 contained components of Artemisia absinthium in abundance. Further, it can also be understood that the more the organic medium is contained in the extracting medium, the more the anti-HIV activity of the extraction liquid is enhanced.

When the Examples 1, 2, and 3 are compared with one another, the liquid extracted by ethanol in the Example 2 exhibited the highest anti-HIV activity. The cytotoxicity of the liquid extracted by ethanol in the Example 2 may be further weakened by using an appropriate method(s).

Further, the inventors also anticipate that sesquiterpenes are active components for the anti-HIV activity. Therefore, it is conceivable to further select an extracting condition(s) for efficiently extracting sesquiterpenes into the extraction liquid. The extract obtained as described above is expected to further improve the degree of the anti-HIV activity. The extract according to this embodiment may be a crudely-purified substance containing, in addition to sesquiterpenes, a component(s) other than the sesquiterpenes.

The above-described extract may be applied, in order to suppress HIV, in industries for developing and manufacturing processed foods, refreshing drinks, health supplemental foods, health supplemental drinks, cosmetics, medicinal cosmetics, hygienic sundry goods, and medicines. The following are examples.
<1> Materials for suppressing the activity of HIV, obtained from Artemisia absinthium.
<2> Processed foods, refreshing drink, health supplemental foods, and health supplemental drinks obtained by using or adding any of the materials of Item <1>.
<3> Cosmetics and medicinal cosmetics obtained by using or adding any of the material of Item <1>.
<4> Hygienic sundry goods obtained by using or adding any of the materials of Item <1>.
<5> Medicines obtained by using or adding any of the materials of Item <1>

This application is based upon and claims the benefit of priority from Japanese patent application No. 2018-168312, filed on August 23, 2018, the disclosure of which is incorporated herein in its entirety by reference.

## Claims

1. A composition containing an extract of Artemisia absinthium, for preventing a human body from being infected with a human immunodeficiency virus.

2. The composition according to Claim 1, wherein the extract is extracted by using an extracting medium containing more than 57.5 vol.% alcohol.

3. The composition according to Claim 2, wherein the extracting medium contains at least one of a monohydric alcohol and a dihydric alcohol as a main component.

4. The composition according to Claim 2, wherein
the extracting medium contains 98 vol.% or more ethanol,
the rest comprises water, and
the extraction is carried out by direct distillation.

5. The composition according to Claim 2, wherein
the extracting medium is 100% of 1,3-butylene glycol, and
the extraction is carried out by leaching.

6. The composition according to any one of Claims 1 to 5, wherein the composition is a microbicide selected from a group consisting of an aqueous solution, a gel, a sol, a cream, and a suppository.

7. The composition according to Claim 6, wherein the composition is one to be applied to a skin or a mucous membrane.

8. The composition according to Claim 6, wherein the composition is one to be applied to a penis of a human, or inside of a vagina or a vulva of a human.

9. An implement comprising a composition according to any one of Claims 1 to 5, the implement being adapted to be applied to a surface of a body.

10. A condom or a dental dam coated with a composition according to any one of Claims 1 to 5.
